# EUROPEAN PATENT SPECIFICATION

(11) **EP 2 979 694 B1**
(45) Date of publication and mention of the grant of the patent: **17.10.2018**
(21) Application number: 14306230.5
(22) Date of filing: 01.08.2014
(51) Int. Cl.: A61K 31/19, A61K 31/198, A61K 31/407, A61K 45/06, A61P 31/04

(54) **Dimercaptosuccinic acid for the treatment of multidrug resistant bacterial infections**
Dimercaptobernsteinsäure zur Behandlung von multiresistenten bakteriellen Infektionen
Acide dimercaptosuccinique pour le traitement des infections bactériennes multirésistantes

(43) Date of publication of application: 03.02.2016
(73) Proprietor: Université de Fribourg, 1700 Fribourg (CH)
(72) Inventor: Nordmann, Patrice, 1700 Fribourg (CH); Poirel, Laurent, 1700 Fribourg (CH); Girlich, Delphine, 94275 Le-Kremlin-Bicêtre (FR)
(74) Representative: Cabinet Plasseraud

(56) References cited:
- WO-A1-2013/032464
- SIEMANN STEFAN ET AL: "Thiols as classical and slow-binding inhibitors of IMP-1 and other binuclear metallo-beta-lactamases.", BIOCHEMISTRY 18 FEB 2003, vol. 42, no. 6, 18 February 2003 (2003-02-18), pages 1673-1683, XP002734140, ISSN: 0006-2960
- VINEETA N ET AL: "Anti microbial activity of dimercaptosuccinic acid (DMSA): a new chelating agent.", JOURNAL OF THE INDIAN SOCIETY OF PEDODONTICS AND PREVENTIVE DENTISTRY DEC 2001, vol. 19, no. 4, December 2001 (2001-12), pages 160-163, XP002734141, ISSN: 0970-4388
- R Gupta et al: "Synthesis, IR Spectral Studies and Antimicrobial Studies of SomeRare Earth Metal Complexes with Meso 2,3-Dimercaptosuccinic Acid", INTERNET , 21 July 2012 (2012-07-21), XP002734142, Retrieved from the Internet: URL:http://download.springer.com/static/pd f/31/art%253A10.1007%252Fs40009-012-0021-2 .pdf?auth66=1420649210_d78e89ce4a6aabd68ac 57f2744eab3ad&ext=.pdf [retrieved on 2015-01-07]
- NAIDU B NARASIMHULU ET AL: "Synthesis, in vitro, and in vivo antibacterial activity of nocathiacin I thiol-Michael adducts.", BIOORGANIC & MEDICINAL CHEMISTRY LETTERS 15 APR 2005, vol. 15, no. 8, 15 April 2005 (2005-04-15) , pages 2069-2072, XP002734143, ISSN: 0960-894X
- TONEY J H ET AL: "Succinic acids as potent inhibitors of plasmid-borne IMP-1 metallo-beta-lactamase.", THE JOURNAL OF BIOLOGICAL CHEMISTRY 24 AUG 2001, vol. 276, no. 34, 24 August 2001 (2001-08-24), pages 31913-31918, XP002734144, ISSN: 0021-9258

## Description

The present invention relates to succinic acid compounds, i.e. dimercaptosuccinic acid, for use in a method of treating a bacterial infection as defined in the present claims.

Current antibacterial chemotherapy for treating multi-drug resistant bacteria is becoming increasingly inadequate due to the rise of clinical resistance, mainly related to the spread of genes encoding β-lactam resistance. β-lactams comprise penicillins, cephalosporins, carbapenems and monobactams, all sharing in common a strained β-lactam ring that acts as a suicide substrate, acylating the key transpeptidase enzymes involved in cell wall formation. Carbapenems which, until now, were the most effective antibiotics for treating Gram-negative infections, are becoming increasingly ineffective. Clinically-available carbapenems worldwide are mostly imipenem, ertapenem and meropenem. Among the β-lactam antibiotics, they possess the broadest spectrum of activity. Those carbapenemases are proteins that degrade not only carbapenems but also many other β-lactams molecules. Their exact spectrum of activity depends of their structure.

Three main classes of clinically-significant carbapenemases have been reported, i.e. Ambler class A, D and B enzymes. Ambler class A and D enzymes are serine proteins whereas class B enzymes are metallo-enzymes.

The above enzymes are named metallo-β-lactamases (MBLs) because they use zinc ion catalysis and water instead of an active serine to hydrolyse the β-lactam ring of β-lactam molecules. A high diversity of MBLs is known, including in particularly NDM, VIM, IMP, SPM, GIM, AIM, and SIM. Those MBLs share a small number of conserved motifs, but otherwise they show significant sequence diversity and have thus been classified into three subclasses: subclass B1 included BcII from *Bacillus cereus,* CfiA from *Bacteroides fragilis,* IMP-1 and VIM-1 from *Pseudomonas aeruginosa,* NDM-1 and relatives (ten variants identified so far) from *Enterobacteriaceae, A. baumannii, P. aeruginosa,* and *blaB* from *Chryseobacterium meningosepticum;* subclass B2 essentially consists of enzymes from *Aeromonas* strains and subclass B3 includes enzymes such as the L1 enzyme from *Stenotrophomonas maltophilia* along with GOB-1 from *Chryseobacterium meningosepticum.* NDM and VIM enzymes have become important in threatening pathogens whereas the other MBLs are spreading relatively locally. Several MBLs have been also identified as natural resistance factors to carbapenems such as in *Stenotrophomonas maltophilia* (another Gram negative species) and in *Flavobacterium* sp.

IMP-type enzymes were first reported in *Enterobacteriaceae* and *Pseudomonas aeruginosa* in Asia (Japan). Then IMP and VIM-types enzymes have been identified worldwide including in Europe. VIM-2 has identified in particularly in *P. aeruginosa* where it remains currently the most widely distributed carbapenemase. Since 2008, NDM enzymes are emerging the frontline of the antibiotic resistance scene. NDM-1. New Delhi metallo-β-lactamase was first identified in an urinary tract isolate of *K. pneumoniae* from a Swedish patient who had been hospitalized in New Delhi in 2008. Subsequently the spread of NDM producers worldwide has been identified rapidly. Several regions may be considered now as endemic for NDM producers such as Indian subcontinent, the Arabic peninsula, the Middle East and the Balkan states. The *bla*_{NDM}-like genes have been identified not only in *Enterobacteriaceae* but also in *A. baumannii* and *P. aeruginosa* in clinical and environmental isolates.

The hydrolytic activity of metallo-enzymes includes all β-lactams except aztreonam. However, many of the MBL producers are also resistant to aztreonam since most of those producers possess additional mechanisms of resistance to β-lactams.

Ten alleles of the *bla*_{NDM} gene are known that sharing 99.4% amino acid identity. Some the NDM proteins (NDM-4, NDM-7) have extended hydrolytic activity towards carbapenems as compared to the reference NDM-1 protein. Similarly, a total of 39 different VIM variants are known.

The drugs of last resort for treating infections due to carbapenemase producers include polymyxins (such as colistin), tigecyline, fosfomycin, and rarely several aminoglycosides. These options are generally limited by a lack of clinical data on efficacy, as well as by concerns about pharmacokinetic and toxicity of several of those molecules.

Colistin is one of the first line agents for treating those infections. Although introduced in the 1950's, its use was abandoned for aminoglycosides. Many data on safety and efficacy of colistin are based therefore on older studies. Neurotoxicity and nephrotoxicity are the two main concerns with colistin. Dosing issue have not been completely clarified and outbreaks of colistin-resistant carbapenemase producers in Enterobacteriaceae have been already reported.

Tigecycline is a new antibiotic of the tetracycline class. Like colistin, there is no oral form of this antibiotic for systemic infections. The main side effect of tigecycline is nausea but pancreatitis has been reported. The efficacy of tigecycline is limited by increasing reports of acquired resistance in vivo and weak tissue diffusion (kidney and urines). It is not therefore recommended for treating pneumonia and urinary tract infections.

Fosfomycin has limited activity against Gram negative systemic infections. In addition, only its oral formula is available in many countries including the USA.

Adding a β-lactamase inhibitor to a β-lactam antibiotic is a highly validated and commercially successful approach to "rescue" an antibiotic failing in the face of emerging resistance and also to extend the activity spectrum to bacterial species not originally susceptible to the original compound. Such combinations were found to be active against many class A β-lactamases (amoxicillin/clavulanate ticarcillin/clavulanate, ampicillin/sulbactam, piperacillin/tazobactam). Attempts to discover a MBL inhibitor drug that my be used safely in human have failed. Most known MBL inhibitors contain a zinc-binding moiety such as narylsulfonylhydrazones, thiomandelic acids, or thiosemicarbazides. MBL inhibition was achieved, but poor selectivity and poor drug-like properties prevented in vitro antimicrobial synergy with a carbapenem. Inhibitors of several MBLs have been identified in the group of dicarboxylic acids such as succinic, maleic and phtlalic acids. Based on a dicarboxylic acid template, they were shown to bind to the zinc atom in the active site of IMP-1. One of those compound which is ME1071, a maleic acid derivative, has completed Phase I clinical trials. ME1071 shows 10- to 100-fold greater affinity than Imipenem or Biapenem for IMP-1 and VIM-2. However, it was recently reported that the affinity of ME1071 for NDM-1 is considerably weaker (Ki = 24mM).

No efficicient treatment is available for treating Gram-negative bacteria producing carbapenemases. Therefore the purpose of the present invention is to contribute to development of antibiotics for treating infections due to multidrug resistant bacteria producing β-lactamases, particularly carbapenemases of the metallo-enzyme class.

Dimercaptosuccinic acid (DMSA), is the compound with the formula HO₂C-CH(SH)-CH(SH-)CO₂H. It has two asymmetric carbons, and can exist as three different stereoisomers. The 2S,3S and 2R,3R isomers are a pair of enantiomers, whereas the 2R,3S isomer is a meso compound. The meso isomer is used as a chelating agent. Dimercaptosuccinic acid is used in the pharmaceutical field as a treatment for heavy metal toxicity.

SIEMANN STEFAN ET AL: "Thiols as classical and slow-binding inhibitors of IMP-1 and other binuclear metallo-beta-lactamases.", BIOCHEMISTRY 18 FEB 2003, vol. 42, no. 6, disclose thiols, such as 20 microM dimercaptosuccinic acid (DMSA), which inhibit IMP-1 of Pseudomonas aeruginosa.

The inventors found that dimercaptosuccinic acid possess very useful original pharmacological properties; it is endowed in particular with remarkable β-lactamase inhibitor properties, more particularly remarkable carbapenemase inhibitor properties. Accordingly, DMSA is able to inhibit the inactivation of beta-lactam antibiotics such that said antibiotics recover anti bacterial properties.

These properties are illustrated below in the experimental section. They justify the use of dimercaptosuccinic acid (DMSA), as β-lactamase, more particularly as carbapenemase inhibitor drugs in antibiotherapy.

Accordingly, the present invention relates to dimercaptosuccinic acid (DMSA) for use in a method of treating bacterial infections, and more particularly in a method of treating infections due to multidrug resistant bacteria producing carbapenemases of the metallo-enzyme class.

The disclosure also relates to the use of dimercaptosuccinic acid for manufacturing a medicament for use as β-lactamase inhibitor or in a method of treating bacterial infections, and more particularly in a method of treating infections due to multidrug resistant bacteria producing β-lactamases, more particularly producing carbapenemases of the MBL class. Bacterial species that will be targeted as MBL producers are in particularly *Enterobacteriaceae, Pseudomonas aeruginosa* and

*Acinetobacter baumannii.*

DMSA is preferably used in a method of treating infections due to multidrug resistant bacteria producing carbapenemases of the metallo-enzyme class.

Of course, dimercaptosuccinic acid acid could be used in association with a suitable β-lactam antibiotic compound, sequentially or preferably concomitantly.

The present disclosure further relates to a method for inhibiting β-lactamases of bacteria in warm-blooded animals, comprising administering to warm-blooded animals a β-lactamase inhibiting effective amount of dimercaptosuccinic acid, more preferably the meso form of dimercaptosuccinic acid.

The disclosure further relates to a method for treating an infection by β-lactamase-producing bacteria in warm-blooded animals, particularly an infection due to multidrug resistant bacteria producing β-lactamases, more particularly producing carbapenemases of the metallo-enzyme class in warm-blooded animals, comprising administering to warm-blooded animals a β-lactamase inhibiting effective amount of dimercaptosuccinic acid, and a β-lactam antibiotic compound sensitive to β-lactamase.

The compounds can be administred orally, rectally, preferably parenterally and the usual daily dose is 30 mg/kg.

For example, intravenous administration of 30 mg/kg daily of DMSA may be useful for treatment of a septicemia due to *Klebsiella pneumoniae* using imipenem (2 g daily) as β-lactam companion molecule.

The antibiotic compound and DMSA may be administered separately or in a single pharmaceutical composition.

These properties also justify the use of dimercaptosuccinic acid in a pharmaceutical composition further comprising a β-lactamase sensitive antibiotic compound.

The antibiotic pharmaceutical compositions of the disclosure are comprised of an effective amount of dimercaptosuccinic acid, preferably DMSA, more preferably the meso form of DMSA, a β-lactam antibiotic compound sensitive to β-lactamase and optionally but preferably an inert pharmaceutical carrier or excipient. The compositions may be, for example in the form of tablets, capsules, granules, or injectable solutions or suspensions.

Examples of suitable excipients are talc, gum arabic, lactose, starch, magnesium stearate, cocoa butter, aqueous and non-aqueous vehicles, fatty substances of animal or vegetable origin, paraffin derivatives, glycols, various wetting, dispersing or emulsifying agents and preservatives.

DMSA may particularly be formulated for parenteral administration (e.g., by injection, for example bolus injection or continuous infusion). The active ingredients may be presented in unit dose form in ampoules, pre-filled syringes small volume infusion or in multi-dose containers with or without an added preservative. Suitable pharmaceutical forms comprise suspensions, solutions, or emulsions in oily or aqueous vehicles. Examples of oily or non-aqueous carriers, diluents solvents or vehicles include propylene glycol, polyethylene glycol, vegetable oils (e.g., olive oil, and injectable organic esters (e.g., ethyl oleate), and may contain adjuvant agents such as preserving, emulsifying or suspending, wetting, stabilizing and/or dispersing agents. The active ingredients may also be in powder form, obtained by aseptic isolation of sterile solid or by lyophilization from solution for constitution before use with a suitable vehicle, such as sterile, pyrogen-free water.

The antibiotic pharmaceutical compositions of the invention are useful for example in the curative treatment of multidrug resistant infections by bacteria such as Enterobacteriaceae (such as *Escherichia coli, Klebsiella pneumoniae, Klebsiella oxytoca, Morganella morganii, Serrratia marcescens, Proteus mirabilis, Enterobacter cloacae, Enterobacter aerogenes*), and Gram-negative non fermenters (such as *Pseudomonas aeruginosa, Stenotrophomonas maltophilia, Acinetobacter baumannii*)

The usual dose, which varies depending on the subject and the infection in question, depends on the nature and the amount of the antibiotic compound (imipenem - meropenem- ertapenem- biapenem - doripenem).

Among the preferred pharmaceutical compositions of the disclosure are those wherein DMSA is associated with one or more β-lactam antibiotic compounds sensitive to β-lactamase, preferably one or more carbapenems, more preferably one or more of the following antibiotic compounds:
- imipenem
- meropenem
- ertapenem
- biapenem
- doripenem
- panipenem
- razupenem
- tebipenem
- lenapenem
- tomopenem, preferably
- imipenem
- meropenem
- ertapenem
- biapenem or
- doripenem
- panipenem, more preferably
- imipenem or
- ertapenem.

Any other β-lactam molecules may be associated with DMSA such as carboxypenicillins (ticarcillin,..) ureidopenicillins (piperacillin...) or cephalosporins (cefotaxime, ceftazidime, cefepime, cefpirome, ceftriaxone) or moxalactam or aztreonam with or without a carbapenem.

In addition, for an enhanced effect, DMSA, and the above combinations of β-lactam molecules with DMSA may further be associated with a polyacid chelating compound, preferably a polyacetic compound, particularly ethylenediaminetetraacetic acid, abbreviated as EDTA. The chelating compounds are preferably used as conjugates such as calcium-ethylenediaminetetraacetate.

The present disclosure also relates to a process for preparing a pharmaceutical composition described above, characterized in that, according to methods known per se, the antibiotic ingredient and DMSA are mixed with optional acceptable excipients, particularly pharmaceutically acceptable excipients.

As previously mentioned, in the present invention, dimercaptosuccinic acid, is preferred, particularly its meso isomer.

The scope of the invention can be understood better by referring to the examples given below, the aim of which is to explain the advantages of the invention.

### EXAMPLES

### EXAMPLE 1: Tablets

Tablets were prepared containing 400 mg of meso dimercaptosuccinic acid, 500 mg of imipenem and sufficient excipient of lactose, starch, talc and magnesium stearate to obtain a final weight of 2 g.

### EXAMPLE 2: Tablets

Tablets were prepared containing 400 mg of meso dimercaptosuccinic acid, 500 mg of ertapenem and sufficient excipient of lactose, starch, talc and magnesium stearate to obtain a final weight of 2 g.

### EXAMPLE 3: Powder for parenteral injections

Vials were prepared containing 400 mg of meso dimercaptosuccinic acid and 500 mg of imipenem. An aqueous solvent was provided in a separate vial.

### EXPERIMENTAL DATA

DMSA and MSA were evaluated as inhibitors of MBLs using a panel of strains producing the most clinically significant carbapenemases of the metallo-enzyme group.

Minimum inhibitory concentrations (MICs) of carbapenems were determined in-vitro with strains (reference and clinical) producing the three main types of class B carbapenemases which are IMP, VIM and NDM, with or without EDTA and DMSA.

Inhibitory properties of DMSA and EDTA were also compared in-vitro by UV spectrophotometry using purified NDM-1 protein and carbapenems as substrates. E. coli TOP10 pCR2.1-(PNDM-1-*bla*NDM-1-*ble*MBL), expressing the *bla*NDM-1 gene with *its original promoter (PNDM-1) cloned into a pTOPO multicopy plasmid and expressed in E. coli* TOP10 reference strain (Dortet *et al.* 2012) was used for preliminary assays in order to determine the most efficient of DMSA being active against NDM producers.

MICs of imipenem was determined for *E. coli* TOP10 pCR2.1-(PNDM-1-*bla*NDM-1-bleMBL) in the presence of 0, 0.01, 0.1 or 1 mg/ml of Ca-EDTA or DMSA by using the broth microdilution method in zinc-complemented M9CA minimum medium (SD7025, Euromedex, France). M9CA minimal medium was used preferably to the standard Mueller Hinton broth in order to determine precisely the concentration of zinc in broth (ZnSO4, Sigma Aldrich, France, 5µg/ml (final concentration).

A 100 mM DMSA stock solution (Sigma Aldrich, France) (pH 5.0) was prepared in 0.2 M NaOH as recommended (Maiorino et al. 1990) and pH was neutralized to pH 7.0 with NaHCO₃ Na₂Ca-EDTA (Sigma Aldrich, France) dissolved in water.

Determination of MIC values of imipenem for *E. coli* TOP10 pCR2.1-(*P*_{NDM-1}- *bla*_{NDM-1}*-ble*_{MBL}) showed that the maximal inhibition efficiency was obtained with 1 mg/ml of DMSA (MIC of imipenem being lowered from 12 to 0.2 µg/ml)

**Table 1**

| Metal chelating agent (mg/ml) | | MIC of imipenem (µg/ml) |
|---|---|---|
| DMSA | 0 | 12 |
| DMSA | 0.01 | 12 |
| DMSA | 0.1 | 12 |
| DMSA | 1 | 0.2 |

One mg/ml of DMSA correspond to 5000 µM. The reported plasma concentrations of DMSA reach ca. 100 µM (Bradberry and Vale, 2009; Asiedu *et al.* 1995) after a single oral dose of DMSA (10 mg). However, (i)) IV administration of DMSA would provide much higher DMSA concentrations in serum (ii) most of the DMSA is rapidly excreted in urines and transformed in vivo in cysteine-DMSA residues (Bradberry and Vale, 2009).

Inhibition of NDM-1 by DMSA was assessed on a purified fraction of NDM-1, obtained as previously described for NDM-4 (Nordmann et al. 2012) with imipenem 100 µM as substrate. The concentration of DMSA suppressing 50% of the NDM-1 hydrolytic activity (IC₅₀) was found to be 1 mM.

Then the concentration of DMSA was chosen for the determination of the MIC values of imipenem and ertapenem for 29 enterobacterial isolates with various levels of MIC of carbapenems, all those isolates possessing β-lactamase genes characterized at the molecular level. The results are reported in Table 2.

The strains were as follows: VIM producers (n=10), IMP producers (n=9), NDM-1 producers (n=10). The selected concentration of DMSA was 10- fold (0.3 mM) and 100-fold (3 mM) that of the human peak serum concentration after oral administration (Asiedu et al;, 1995), and that of Ca-EDTA was assayed at the fixed concentration of 0.2 mM (Table 2). The MICs of carbapenems varied widely among isolates producing a same carbapenemase type. DMSA at 3 mM lowered the MICs of imipenem and ertapenem of IMP-like producers (100% of the tested isolates), and VIM-like producers (70 to 80 % of the tested isolates). DMSA at 3 mM reduced the MICs of ertapenem of NDM-like producers for 50 % of the tested isolates, but did not significantly affect the MIC values of imipenem for those isolates. The lowest concentration of DMSA tested (0.3 mM) lowered the MICs of carbapenems for most IMP-like producing isolates, but not for those producing other MBLs (Table 2).

**Table 2**

| Strains | beta-lactamase content | | | | | | |
|---|---|---|---|---|---|---|---|
| | | IPM | IPM + DMSA 0.3mM | IPM + DMSA 3 mM | ETP | ETP+ DMSA 0.3mM | ETP+ DMSA 3mM |
| *K.p.* 0709124 | **IMP-1b** + TEM-15 | 32 | 2 | 1 | 32 | 8 | 0.06 |
| *K.p. 6852* | **IMP-1** + SHV-5 | >32 | 32 | 1 | >32 | >32 | 16 |
| *E. coli* 1108013 | **IMP-1** + TEM-1 | 4 | 1 | 0.25 | 32 | 1 | 0.12 |
| *E. coli* JAP | **IMP-1** | 0.25 | 0.25 | 0.25 | 0.5 | 0.12 | 0.03 |
| *E. cloacae* 1008186 | **IMP-1** | 16 | 2 | 2 | 32 | 32 | 2 |
| *E. cloacae* 1008183 | **IMP-1** | 8 | 2 | 2 | 8 | 1 | 0.25 |
| *E. cloacae* 1008085 | **IMP-1** | 2 | 0.12 | 0.25 | 8 | 2 | 1 |
| *K.p.* TAW | **IMP-8** + SHV-5 | 4 | 1 | 1 | 0.5 | 0.12 | 0.12 |
| *E. cloacae* TAW | **IMP-8** | 4 | 2 | 1 | 4 | 2 | 0.25 |
| *K.p. ROUM* | **VIM** | 32 | 32 | 16 | >32 | > 32 | 4 |
| *E. coli* 1008077 | **VIM-1** + TEM-3 | 32 | 32 | 16 | >32 | 32 | 2 |
| *E. coli* 0404018 | **VIM-1** + CMY-6 | 4 | 4 | 2 | 4 | 2 | 0.12 |
| *E. coli* MAD | **VIM-1** + CTX-M-3 | 0.25 | 0.25 | 0.06 | 0.25 | 0.12 | 0.06 |
| *E. cloacae* 1008029 | **VIM-1** + CTX-M-3 | 16 | 16 | 4 | >32 | >32 | 32 |
| *E. cloacae 396715* | **VIM-1** | 4 | 4 | 2 | 2 | 2 | 0.5 |
| *C*. *freundii MIG* | **VIM-2** + TEM-1 +OXA-9 + OXA-10 | 1 | 0.5 | 1 | 2 | 2 | 2 |
| *E. coli* IBIS | **VIM-4** | 1 | 1 | 1 | 2 | 1 | 0.12 |
| *E. cloacae* KOW3 | **VIM-1** + CTX-M-15+ TEM-1 + SHV-31 | 32 | 32 | 8 | >32 | >32 | 32 |
| *E. coli DIH* | **VIM-19** | 16 | 8 | 4 | 8 | 16 | 2 |
| *K.p.* OMA1 | **NDM-1** + TEM-1 +CTX-M-15+ SHV-28 + OXA-1 + OXA-9 | 32 | >32 | 32 | >32 | >32 | >32 |
| *K.p.* KIE | **NDM-1** + SHV-38 + CMY-16 + OXA-10 | 4 | 4 | 4 | 4 | 4 | 1 |
| *E. coli* RIC | **NDM-1** + CMY-16 + OXA-1 + OXA-10 | 4 | 4 | 4 | 8 | 8 | 0.5 |
| *E. coli* IR5 | **NDM-1 +** TEM-1 +CTX-M-15 | 2 | 2 | 1 | 32 | 16 | 4 |
| *E. coli* ALL | **NDM-1** + TEM-1 +CTX-M-15+OXA-1 + OXA-2 | 1 | 1 | 0.5 | 16 | 16 | 2 |
| *E. coli* BOUN | **NDM-4** + OXA-1 | 2 | 2 | 1 | 16 | 16 | 4 |
| *E. coli* FEK | **NDM-4** + NDM-4 + OXA-1 + CTX-M-15 | 32 | 32 | 8 | >32 | >32 | >32 |
| *E. coli* 405 | **NDM-5** + TEM-1 +CTX-M-15 | 32 | 16 | 8 | >32 | >32 | >32 |
| *E. coli* 162 | **NDM-5** + TEM-1 +CTX-M-15 | 32 | 32 | 32 | >32 | >32 | >32 |
| *E. coli* COUP | **NDM-7** + OXA-1 +CTX-M-15 | 2 | 2 | 2 | 32 | 32 | 16 |

| | | | | | | | |
|---|---|---|---|---|---|---|---|
| Abbreviations : K.p.= *K. pneumoniae,* IMP= imipenem ; ETP= ertapenem; DMSA= meso-2,3-dimercaptosuccinic acid, Bolded names correspond to those of MBLs. | | | | | | | |

DMSA reduced the MICs of ertapenem for bacteria producing NDM-1 though the synergy effect was weaker than that observed for bacteria producing IMP and VIM-type MBLs. In addition DMSA and MSA efficacy was studied in association with meropenem that showed even more promising results (Table 3).

MICs of meropenem with of without DMSA or MSA

**Table 3**

| | MIC of meropenem (µg/ml) | | |
|---|---|---|---|
| | | DMSA | MSA |
| *Kp* **NDM** KIE | 4 | 0.5 | 0.19 |
| *Kp* **NDM** IND | 4 | 4 | 1 |
| *E. coli* **NDM** CHAN | 3 | 2 | 0.12 |
| *E. coli* **NDM** FEK | 6 | 4 | 0.25 |
| *E. coli* **NDM** pCR2.1 | > 32 | 8 | 1 |
| *Kp* **VIM** KOW1 | > 32 | 32 | 1.5 |
| *E. coli* **VIM** KOW7 | 0.5 | 0.25 | 0.03 |
| *Kp* C3 KPC-3 | > 32 | > 32 | > 32 |

DMSA and MSA were used at a concentration of 3 mM each. A negative control was used as strain KpC3 producing the non- metallo carbapenemase KPC

In conclusion, DMSA allows treating mammalian, particularly human infections due to bacteria producing at least of several types of metallo-β-lactamases.

Compounds DMSA or MSA as shown in Tables 2/3, have the effect of recovering the antibacterial activities of imipenem and ertapenem against MBL-producing bacteria of which therapy has hitherto been regarded difficult in the medical field. Thus, DMSA and MSA are useful as MBL inhibitors for the combination with β-lactam drugs.

## Claims

1. Dimercaptosuccinic acid (DMSA) for use in a method of treating a bacterial infection due to one or more multidrug resistant bacteria producing carbapenemases of the metallo-enzyme class, wherein DMSA is administered at a dose of 30 mg/kg.

2. A compound for use according to claim 1, **characterized in that** the compound is the meso form of dimercaptosuccinic acid.

## Patentansprüche

1. Dimarcaptosuccinsäure (DMSA) zur Verwendung in einem Verfahren zur Behandlung einer bakteriellen Infektion aufgrund eines oder mehrerer multiresistenter Bakterien, die Carbapenemasen der Metalloenzym-Klasse bilden, worin DMSA mit einer Dosis von 30 mg/kg verabreicht wird.

2. Verbindung nach Anspruch 1, **dadurch gekennzeichnet, dass** die Verbindung in der meso-Form von Dimercaptosuccinsäure vorliegt.

## Revendications

1. Acide dimercaptosuccinique (DMSA) pour son utilisation dans un procédé de traitement d'une infection bactérienne due à une ou plusieurs bactéries multirésistantes produisant des carbapénémases de la classe des métallo-enzymes, dans lequel le DMSA est administré à une dose de 30 mg/kg.

2. Composé pour son utilisation selon la revendication 1, **caractérisé en ce que** le composé est la forme méso de l'acide dimercaptosuccinique.
